# EUROPÄISCHE PATENTSCHRIFT

(11) **EP 2 276 404 B1**
(45) Veröffentlichungstag und Bekanntmachung des Hinweises auf die Patenterteilung: **26.10.2011**
(21) Anmeldenummer: 09737833.5
(22) Anmeldetag: 21.04.2009
(51) Int. Cl.: A61B 5/107, G01B 3/10

(54) **ROLLMASSBAND**
FLEXIBLE MEASURING TAPE
RUBAN DE MESURE À ENROULEMENT

(30) Priorität: 30.04.2008 DE 102008021821
(43) Veröffentlichungstag der Anmeldung: 26.01.2011
(73) Patentinhaber: Ottleben, Michael, 37589 Kalefeld (DE)
(72) Erfinder: Ottleben, Michael, 37589 Kalefeld (DE)
(74) Vertreter: Callies, Rainer Michael
(86) Internationale Anmeldenummer: PCT/EP2009/002887
(87) Internationale Veröffentlichungsnummer: WO 2009/132789

(56) Entgegenhaltungen:
- JP-A- 6 042 901
- US-A1- 2007 271 806

## Beschreibung

Die Erfindung betrifft ein Rollmaßband nach dem Oberbegriff des Anspruchs 1. Dabei ist das Rollmaßband zur Verwendung im Gesundheitswesen, z.B. in Krankenhäusern, Sanitätshäusern, Arztpraxen, usw., vorgesehen, um Längen- bzw. Umfangsmessungen an Personen vorzunehmen. Ferner bezieht sich die Erfindung auf Zusatzausstattungsmittel für ein Rollmaßband nach dem Oberbegriff des Anspruchs 1.

Im Bereich des Gesundheitswesens gibt es selbstverständlich vielfältige Anlässe, Körperabmessungen von Personen bestimmen zu müssen. Wenn hierzu ein Maßband bzw. Messband verwendet wird, wird dieses häufig Kontakt mit der Haut haben müssen. Bei einer mehrfachen Verwendung eines Maßbandes ergeben sich somit hygienische Probleme, wenn das Maßband zwischen den einzelnen Verwendungen nicht ausreichend desinfiziert wird. Eine hinreichende Desinfektion ist jedoch aufwändig bzw. insbesondere bei Rollmaßbändern, die sich nämlich durch ein sich selbst aufrollendes Messband auszeichnen (siehe z.B. DT 25 45 203 B1), kaum praktikabel. Dies ist beispielsweise in der DE 20 2005 008 912 U1 ausgeführt, die eine die hygienischen Probleme lösende Vorrichtung zur Ermittlung und Anzeige der passgenauen Größe eines Thrombosestrumpfes zum Gegenstand hat.

Auch die DE 101 63 745 A1, die ein Verfahren zur bereichsweisen Erfassung einer Außenkontur eines Körpers betrifft, spricht das Hygieneproblem bei der Verwendung von Maßbändern an.

Aus der JP 06 042901 A ist ein Maßband bekannt, das eine Reinigungsvorrichtung zum zwangsläufigen Reinigen eines zugehörigen Messbandes aufweist. Dabei wird das Messband beim Heraus- und Zurückziehen entlang und in Kontakt mit einem weichen Material geführt, welches an einer Öffnung eines das Messband enthaltenden Behälters angeordnet ist. Eine Desinfektion des Messbandes ist dabei nicht vorgesehen.

Die DE 1 978 004 U betrifft einen Messstab zum Messen der Größe von Säuglingen und Kleinstkindern. Der Messstab ist aus Kunststoff gespritzt, welcher sich im Heißluftstrom keimfrei machen lässt.

Die US 2007/0271806 A1 beschreibt ein flexibles, selbstklebendes Maßband, das auch zur Verwendung im medizinischen Bereich vorgesehen ist. Das Problem einer mikrobiellen Verunreinigung wird dadurch angegangen, dass zur Herstellung des Maßbandes antibaktieriell behandelte Klebstoffe, die steril und verunreinigungsfrei sind, verwendet werden. Dies ist jedoch eine speziell aufgrund der Selbstklebeeigenschaft des Maßbandes mögliche Lösung des hygienischen Problems.

Der Erfindung liegt daher die Aufgabe zugrunde, ein gattungsgemäßes Rollmaßband zur Verfügung zu stellen, das auf einfache Weise eine Desinfektion des Messbandes ermöglicht. Ferner liegt der Erfindung die Aufgabe zugrunde, Zusatzausstattungsmittel für ein gattungsgemäßes Rollmaßband zur Verfügung zu stellen, die es erlauben, das Rollmaßband nachträglich so auszustatten, dass eine einfache Desinfektion möglich ist.

Die erstgenannte Aufgabe wird durch die Merkmale des Anspruchs 1 gelöst. Das Rollmaßband weist ein Gehäuse und ein Messband mit einer Längenmaßeinteilung auf. Das Rollmaßband besitzt eine Aufrollvorrichtung, die dafür sorgt, dass das Messband innerhalb des Gehäuses aufrollbar ist. Solche Aufrollvorrichtungen weisen in der Regel eine Feder und eine Rücklaufsperre auf und sind dem Fachmann bekannt (siehe z.B. DT 25 45 203 B1), so dass auf deren Ausgestaltung hier nicht näher eingegangen wird. Das Gehäuse weist eine Öffnung zum Hindurchführen des Messbandes auf. Es kann insbesondere vorgesehen sein, dass das Messband im aufgerollten Zustand mit einem Anfangsende etwas aus der Gehäuseöffnung herausragt, um von einem Benutzer mit den Fingern gegriffen und herausgezogen werden zu können. Das Rollmaßband weist einen Behälter auf, der zur Aufnahme von flüssigem Desinfektionsmittel vorgesehen ist. Ferner besitzt das Rollmaßband Mittel zum Auftragen von Desinfektionsmittel, das aus dem Behälter stammt, auf zumindest eine Oberfläche des Messbandes, wenn dieses durch die Gehäuseöffnung hindurch bewegt wird. Bei der Bewegung des Messbandes kann es sich um ein Herausziehen und/oder ein Einziehen bzw. Aufrollen nach der Benutzung handeln. Wenn das Messband flach ausgebildet ist und somit zwei Oberflächen aufweist, was in der Regel der Fall ist, kann insbesondere vorgesehen sein, dass dabei beide Oberflächen durch die Auftragungsmittel mit Desinfektionsmittel versehen werden.

Vorzugsweise weisen die Auftragungsmittel zumindest einen Körper auf, der derartig angeordnet ist, dass der Körper bei einem Bewegen des Messbandes durch die Öffnung hindurch das Messband berührt. Vorzugsweise ist der Körper benachbart zu der Gehäuseöffnung angeordnet. Dabei ist der Körper aus einem flüssigkeitsaufsaugenden Material hergestellt und in Fließverbindung mit dem Behälter. Das heißt, es ist vorgesehen, dass in dem Behälter enthaltenes Desinfektionsmittel zu dem Körper gelangen kann. Dies kann insbesondere durch die Saugwirkung des Körpers geschehen oder auch dadurch, dass das Rollmaßband durch den Benutzer so gehalten werden kann, dass durch Unterstützung durch die Schwerkraft Desinfektionsmittel aus dem Behälter zu dem Körper fließt.

Vorzugsweise weisen die Auftragungsmittel zwei solche Körper auf, die einander gegenüberliegen. Auf diese Weise ist es möglich, dass die beiden Körper beim Bewegen des Messbandes zwischen ihnen hindurch aufgrund Berührung des Messbandes Desinfektionsmittel durch Abstreifung auf zwei Messbandoberflächen, die in der Regel eben ausgestaltet sind, auftragen.

Vorzugsweise ist vorgesehen, dass solch ein Auftragungskörper ein Versorgungsröhrchen umgibt, insbesondere vollständig umschließt. Das Versorgungsröhrchen weist mindestens eine Eintrittsöffnung auf, die in den Desinfektionsmittel-Behälter hineinragt, und ferner eine oder mehrere Austrittsöffnungen, die vorzugsweise an der Umfangsfläche des Versorgungsröhrchens angeordnet sind. Diese Austrittsöffnungen können insbesondere im Wesentlichen über einen gesamten aus dem Behälter herausragenden Längenabschnitt des Versorgungsröhrchens angeordnet sein. Der Auftragungskörper liegt an der Umfangsfläche des Versorgungsröhrchens an, so dass Desinfektionsmittel aus den Austrittsöffnungen in den Auftragungskörper gelangen kann.

Bei dem Auftragungskörper kann es sich zumindest teilweise um einen Schwamm oder Filz handeln. Beide Materialien besitzen eine Saugfähigkeit, die es ermöglicht, Desinfektionsmittel an- bzw. aufzusaugen und aufgrund ihrer Verformbarkeit effektiv auf das Messband abzustreifen. Insbesondere kann der Auftragungskörper eine bzw. zwei gegenüberliegende Filzlippen aufweisen.

Der Behälter kann außen an dem Gehäuse befestigt sein, oder er kann in das Gehäuse integriert sein, d.h. sich integral an dem Gehäuse oder innerhalb des Gehäuses befinden. Ferner kann der Behälter eine Öffnung zum Nachfüllen von Desinfektionsmittel aufweisen.

Der Behälter kann zumindest teilweise eine verformbare Außenwandung aufweisen, so dass durch einen auf diese ausgeübten Druck das Behältervolumen verringert und dadurch der Innendruck im Behälter erhöht werden kann. Auf diese Weise kann es erleichtert werden, dass eine noch im Behälter enthaltene Restmenge an Desinfektionsmittel durch den so erzeugten Überdruck aus dem Behälter austreten kann. Solch ein Druck kann, wenn der Behälter entsprechend zugänglich angeordnet ist, also insbesondere wenn er außen am Gehäuse befestigt ist, durch einen Benutzer bei Bedarf ausgeübt werden.

Die zweitgenannte Aufgabe wird durch die Merkmale des Anspruchs 9 gelöst. Die Zusatzausstattungsmittel sind so beschaffen, dass sie zur nachträglichen Ausstattung eines in dem Anspruch genannten Rollmaßbandes geeignet sind. Durch eine solche nachträgliche Ausstattung entsteht ein Rollmaßband gemäß Anspruch 1, so dass hier auf die obigen diesbezüglichen Erläuterungen Bezug genommen wird. Die Zusatzausstattungsmittel bzw. die Zusatzvorrichtung, die bei Anbringung der Zusatzausstattungsmittel an einem Rollmaßband oder bei Zusammensetzung der Zusatzausstattungsmittel entsteht, sind deshalb sehr vorteilhaft, weil mit ihnen vorhandene herkömmliche Rollmaßbänder weiterverwendet werden können, ohne dabei auf die erfindungsgemäße Desinfektionsmöglichkeit verzichten zu müssen.

Bevorzugte Ausführungsformen der Zusatzausstattungsmittel sind in den Ansprüche 10 bis 15 beschrieben. Der Auftragungskörper könnte, wenn die Zusatzausstattungsmittel an einem herkömmlichen Rollbandmaß befestigt sind, auch an einer anderen Stelle als benachbart zu der Gehäuseöffnung angeordnet sein. Ferner kann die mindestens eine Austrittsöffnung des Versorgungsröhrchens auch an einer anderen Stelle als an der Umfangsfläche des Versorgungsröhrchens angeordnet sein.

Die oben in Bezug auf das erfindungsgemäße Rollmaßband genannten Vorteile gelten auch bei den hier genannten Ausführungsformen der Zusatzausstattungsmittel bzw. der aus ihnen zusammengesetzten Zusatzvorrichtung entsprechend.

Ferner kann insbesondere vorgesehen sein, dass der Behälter der Zusatzausstattungsmittel außen an dem Gehäuse eines herkömmlichen Rollmaßbandes zu befestigen ist. Dazu kann als das Befestigungsmittel der Zusatzausstattungsmittel ein Flachteil vorgesehen sein, das auf einer Oberfläche des Behälters angebracht ist und eine vorzugsweise selbstklebende freie Oberfläche aufweist, auf der das Rollmaßband mit seinem Gehäuse befestigt werden kann.

Der Behälter der Zusatzausstattungsmittel kann eine Nachfüllöffnung aufweisen.

Die oben in Bezug auf das erfindungsgemäße Rollmaßband beschriebene Ausgestaltung des Behälters mit einer verformbaren Außenwandung kann entsprechend auch bei dem Behälter der Zusatzausstattungsmittel vorgesehen sein.

Im Folgenden wird die Erfindung anhand eines Ausführungsbeipiels näher erläutert, wobei auf die Figuren Bezug genommen wird. Es zeigen:

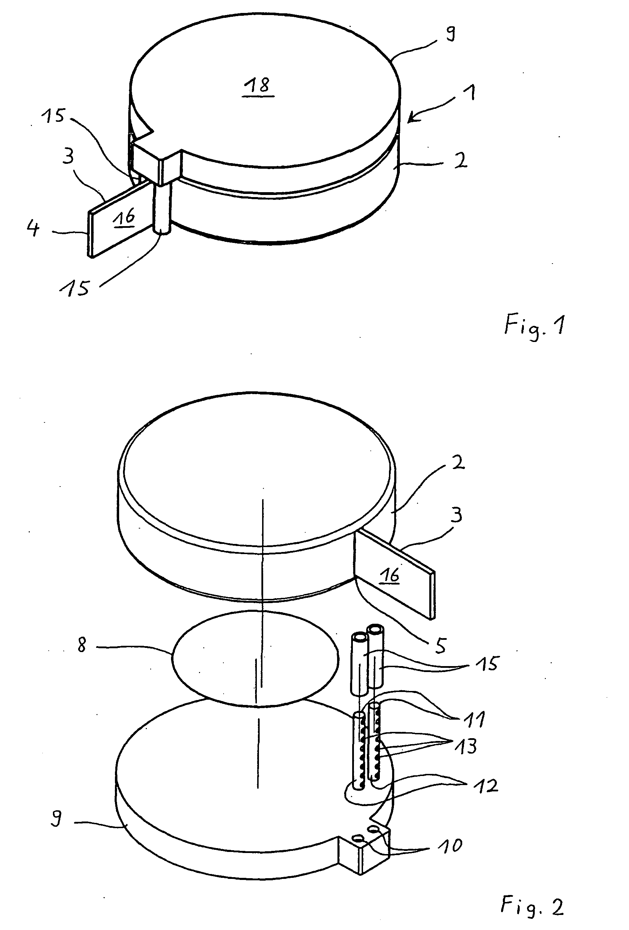

Fig. 1 ein erfindungsgemäßes Rollmaßband, das durch Anbringung von erfindungsgemäßen Zusatzausstattungsmitteln an einem herkömmlichen Rollmaßband entstanden ist,
Fig. 2 das Rollmaßband gemäß Fig. 1 in um 180° gedrehter Position und auseinandergezogener Darstellung.

Das erfindungsgemäße Rollmaßband ist mit dem Bezugszeichen 1 bezeichnet. Es weist ein Gehäuse 2 auf, in dem sich ein Messband 3 in aufgerolltem Zustand befindet. Ein Anfangsende 4 des Messbandes 3 ragt zusammen mit einem herausgezogenen Messbandabschnitt aus einer Öffnung 5 des Gehäuses 2 heraus.

An dem Gehäuse 2 ist mittels eines als Klebepad bezeichneten, selbstklebenden Flachteils 8 ein Behälter 9 für ein flüssiges Desinfektionsmittel befestigt, der auch als Tank bezeichnet werden könnte. Der Behälter 9 weist zwei Öffnungen 10 auf, in die jeweils ein Versorgungsröhrchen 11 gesteckt ist. Die beiden Versorgungsröhrchen 11 weisen jeweils an einer Umfangsfläche 12 zwei sich gegenüberliegende Reihen (jeweils nur eine ist zu sehen) von Löchern 13 auf. Einige dieser Löcher 13 sind innerhalb des Inneren des Behälters 9 als Eintrittsöffnungen angeordnet, so dass Desinfektionsmittel aus dem Behälter 9 in die Versorgungsröhrchen 11 gelangen kann. Die weiteren Löcher 13 der Versorgungsröhrchen 11 dienen als Austrittsöffnungen und sind von einem anliegenden röhrenförmigen Schwamm 15 umgeben. Die Versorgungsröhrchen 11 sind jeweils an dem oberen und dem unteren Ende geschlossen. Die Behälteröffnungen 10 können auch zum Nachfüllen von Desinfektionsmittel in den Behälter 9 dienen, wozu von diesem die Versorgungsröhrchen 11 mit den Schwämmen 15 abgezogen werden können.

Das Messband 3 hat nahe bzw. benachbart zu der Gehäuseöffnung 5 Kontakt mit beiden Schwämmen 15. Diese geben Desinfektionsmittel (nicht gezeigt), das von dem Behälter 9 über die Versorgungsröhrchen 11 durch Saugwirkung und/oder durch Schwerkrafteinwirkung in sie hineingelangt ist, an zwei gegenüberliegende Messband-Oberflächen 16 mit nicht gezeigter Längenmaßeinteilung ab. Auf diese Weise findet eine Desinfektion des Messbandes 3 sowohl bei einem Herausziehen aus dem Gehäuse 2 als auch einem Aufrollen nach einer Benutzung statt. Zum Aufrollen ist im Gehäuse 3 ein dem Fachmann bekannter, eine Feder aufweisender Mechanismus (nicht gezeigt) angeordnet.

Ein mit dem Bezugszeichen 18 bezeichneter Außenwandungsbereich des Behälters 9 kann derartig verformbar bzw. flexibel sein, dass er bei Ausübung eines Drucks durch einen Benutzer mit einem Finger etwas eingedrückt werden kann, um dadurch den Druck innerhalb des Behälters zu erhöhen und ein Austreten von Desinfektionsmittel aus dem Behälter 9 zu erleichtern. Dies ist dann vorteilhaft, wenn nur noch eine relativ geringe Menge an Desinfektionsmittel in diesem enthalten ist.

Erfindungsgemäße Zusatzausstattungsmittel sind durch die Gesamtheit der Bauteile Behälter 9, Versorgungsröhrchen 11 mit Schwämmen 15 und Klebepad 8 gegeben. Solche Zusatzausstattungsmittel bzw. eine aus ihnen zusammengesetzte Zusatzvorrichtung können - wie in diesem Ausführungsbeispiel - auf einfache Weise nachträglich an einem herkömmlichen Rollmaßband, wie es beispielhaft in den Figuren mit Gehäuse 2 und Messband 3 gezeigt ist, befestigt werden.

## Patentansprüche

1. Rollmaßband (1) zur Längen- und Umfangsmessung, aufweisend ein Gehäuse (2) und ein Messband (3) mit einer Längenmaßeinteilung, welches innerhalb des Gehäuses (2) mittels einer Aufrollvorrichtung aufrollbar ist und durch eine Öffnung (5) des Gehäuses (2) geführt werden kann,
**dadurch gekennzeichnet, dass** das Rollmaßband (1) einen Behälter (9) für ein flüssiges Desinfektionsmittel aufweist und Auftragungsmittel (11,15), die so ausgelegt sind, dass, wenn der Behälter (9) ein Desinfektionsmittel enthält, bei einem Bewegen des Messbandes (3) durch die Gehäuseöffnung (5) hindurch ein Teil des Desinfektionsmittels auf zumindest eine Oberfläche (16) des Messbandes (3) aufgetragen wird.

2. Rollmaßband (1) nach Anspruch 1,
**dadurch gekennzeichnet, dass** die Auftragungsmittel zumindest einen Körper (15) aufweisen, der benachbart zu der Gehäuseöffnung (5) so angeordnet ist, dass er bei einem Bewegen des Messbandes (3) durch die Gehäuseöffnung (5) hindurch das Messband (3) berührt, und der aus einem flüssigkeitsaufsaugenden Material hergestellt und in Fließverbindung mit dem Behälter (9) ist.

3. Rollmaßband (1) nach Anspruch 2,
**dadurch gekennzeichnet, dass** die Auftragungsmittel (11,15) zwei solche Körper (15) aufweisen, die einander gegenüberliegen.

4. Rollmaßband (1) nach Anspruch 2 oder 3,
**dadurch gekennzeichnet, dass** der Körper (15) ein Versorgungsröhrchen (11) umgibt, das zumindest eine in den Behälter (9) hineinragende Eintrittsöffnung (13) und zumindest eine an einer Umfangsfläche (12) angeordnete Austrittsöffnung (13) aufweist.

5. Rollmaßband (1) nach einem der Ansprüche 2 bis 4,
**dadurch gekennzeichnet, dass** der Körper einen Schwamm (15) aufweist.

6. Rollmaßband (1) nach einem der Ansprüche 2 bis 5,
**dadurch gekennzeichnet, dass** der Körper (15) Filz aufweist.

7. Rollmaßband (1) nach Anspruch 6,
**dadurch gekennzeichnet, dass** der Körper (15) eine Filzlippe aufweist.

8. Rollmaßband (1) nach einem der vorhergehenden Ansprüche,
**dadurch gekennzeichnet, dass** der Behälter (9) außen an dem Gehäuse (2) befestigt ist.

9. Zusatzaustattungsmittel für ein Rollmaßband, das zur Längen- und Umfangsmessung ausgelegt ist und ein Gehäuse (2) und ein Messband (3) mit einer Längenmaßeinteilung, welches innerhalb des Gehäuses (2) mittels einer Aufrollvorrichtung aufrollbar ist und durch eine Öffnung (5) des Gehäuses (2) geführt werden kann, aufweist,
wobei die Zusatzaustattungsmittel einen Behälter (9) für ein flüssiges Desinfektionsmittel, ein Befestigungsmittel (8) zur Befestigung des Behälters (9) an dem Gehäuse (2) und Auftragungsmittel (11,15) aufweisen, die so ausgelegt sind, dass, wenn die Zusatzausstattungsmittel an dem Rollbandmaß angebracht sind und der Behälter (9) ein Desinfektionsmittel enthält, bei einem Bewegen des Messbandes (3) durch die Gehäuseöffnung (5) hindurch ein Teil des Desinfektionsmittels auf zumindest eine Oberfläche (16) des Messbandes (3) aufgetragen wird.

10. Zusatzaustattungsmittel nach Anspruch 9,
**dadurch gekennzeichnet, dass** die Auftragungsmittel (11,15) zumindest einen Körper (15) aufweisen, der bei Befestigung der Zusatzausstattungsmittel an dem Rollmaßband benachbart zu der Gehäuseöffnung (5) so angeordnet ist, dass er bei einem Bewegen des Messbandes (3) durch die Gehäuseöffnung (5) hindurch das Messband (3) berührt, und der aus einem flüssigkeitsaufsaugenden Material hergestellt und in Fließverbindung mit dem Behälter (9) ist.

11. Zusatzaustattungsmittel nach Anspruch 10,
**dadurch gekennzeichnet, dass** die Auftragungsmittel (11,15) zwei solche Körper (15) aufweisen, die einander gegenüberliegen.

12. Zusatzaustattungsmittel nach Anspruch 10 oder 11,
**dadurch gekennzeichnet, dass** der Körper (15) ein Versorgungsröhrchen (11) umgibt, das zumindest eine in den Behälter (9) hineinragende Eintrittsöffnung (13) und zumindest eine an einer Umfangsfläche (12) angeordnete Austrittsöffnung (13) aufweist.

13. Zusatzaustattungsmittel nach einem der Ansprüche 10 bis 12,
**dadurch gekennzeichnet, dass** der Körper einen Schwamm (15) aufweist.

14. Zusatzaustattungsmittel nach einem der Ansprüche 10 bis 13,
**dadurch gekennzeichnet, dass** der Körper (15) Filz aufweist.

15. Zusatzaustattungsmittel nach Anspruch 14,
**dadurch gekennzeichnet, dass** der Körper (15) eine Filzlippe aufweist.

## Claims

1. Flexible tape measure (1) for measuring length and circumference, comprising a housing (2) and a measuring tape (3) having a linear scale which can be rolled up inside the housing (2) by means of a roll-up device and can be guided through an opening (5) in the housing (2),
**characterised in that** the flexible tape measure (1) comprises a container (9) for a liquid disinfectant and application means (11, 15) which are designed such that when the container (9) contains a disinfectant, a portion of the disinfectant is applied to at least one surface (16) of the measuring tape (3) as the measuring tape (3) moves through the housing opening (5).

2. Flexible tape measure (1) as claimed in claim 1,
**characterised in that** the application means comprise at least one body (15) which is disposed adjacent to the housing opening (5) such that it contacts the measuring tape (3) as the measuring tape (3) moves through the housing opening (5), and which is produced from a liquid-absorbent material and is in flow-connection with the container (9).

3. Flexible tape measure (1) as claimed in claim 2,
**characterised in that** the application means (11, 15) comprise two such bodies (15) which are opposite one another.

4. Flexible tape measure (1) as claimed in claim 2 or 3,
**characterised in that** the body (15) surrounds a supply tube (11) which comprises at least one inlet opening (13), which protrudes into the container (9), and at least one outlet opening (13) which is disposed on a peripheral surface (12).

5. Flexible tape measure (1) as claimed in any one of claims 2 to 4,
**characterised in that** the body comprises a sponge (15).

6. Flexible tape measure (1) as claimed in any one of claims 2 to 5,
**characterised in that** the body (15) comprises felt.

7. Flexible tape measure (1) as claimed in claim 6,
**characterised in that** the body (15) comprises a felt lip.

8. Flexible tape measure (1) as claimed in any one of the preceding claims, **characterised in that** the container (9) is attached externally to the housing (2).

9. Supplementary equipment means for a flexible tape measure which is designed for measuring length and circumference and comprises a housing (2) and a measuring tape (3) having a linear scale, which measuring tape can be rolled up inside the housing (2) by means of a roll-up device and can be guided through an opening (5) in the housing (2),
wherein the supplementary equipment means comprise a container (9) for a liquid disinfectant, an attachment means (8) for attaching the container (9) to the housing (2), and application means (11, 15) which are designed in such a manner that when the supplementary equipment means are attached to the flexible tape measure and the container (9) contains a disinfectant, a portion of the disinfectant is applied to at least one surface (16) of the measuring tape (3) as the measuring tape (3) moves through the housing opening (5).

10. Supplementary equipment means as claimed in claim 9,
**characterised in that** the application means (11, 15) comprise at least one body (15) which, when the supplementary equipment means are attached to the flexible tape measure, is disposed adjacent to the housing opening (5) such that the body contacts the measuring tape (3) as the measuring tape (3) moves through the housing opening (5), and which is produced from a liquid-absorbent material and is in flow-connection with the container (9).

11. Supplementary equipment means as claimed in claim 10,
**characterised in that** the application means (11, 15) comprise two such bodies (15) which are opposite one another.

12. Supplementary equipment means as claimed in claim 10 or 11,
**characterised in that** the body (15) surrounds a supply tube (11) which comprises at least one inlet opening (13), which protrudes into the container (9), and at least one outlet opening (13) which is disposed on a peripheral surface (12).

13. Supplementary equipment means as claimed in any one of claims 10 to 12, **characterised in that** the body comprises a sponge (15).

14. Supplementary equipment means as claimed in any one of claims 10 to 13, **characterised in that** the body (15) comprises felt.

15. Supplementary equipment means as claimed in claim 14,
**characterised in that** the body (15) comprises a felt lip.

## Revendications

1. Mètre ruban à enroulement (1) pour mesurer des longueurs et des circonférences, comportant un boîtier (2) et un ruban de mesure (3) avec une graduation en unités de longueur, qui peut être enroulé à l'intérieur du boîtier (2) au moyen d'un dispositif d'enroulement et qui peut être guidé à travers une ouverture (5) du boîtier (2),
**caractérisé en ce que** le mètre ruban à enroulement (1) comporte un réceptacle (9) pour un désinfectant liquide et des moyens d'application (11, 15) qui sont conçus de telle sorte que, lorsque le réceptacle (9) contient un désinfectant, une partie du désinfectant est appliquée sur au moins une surface (16) du ruban de mesure (3) au moment où le ruban de mesure (3) passe à travers l'ouverture (5) du boîtier.

2. Mètre ruban à enroulement (1) selon la revendication 1, **caractérisé en ce que** les moyens d'application comportent au moins un corps (15), qui est disposé à proximité de l'ouverture (5) du boîtier de telle sorte qu'il vient en contact avec le ruban de mesure (3) au moment où le ruban de mesure (3) passe à travers l'ouverture (5) du boîtier, et qui est réalisé dans un matériau absorbant le liquide et est en liaison fluidique avec le réceptacle (9).

3. Mètre ruban à enroulement (1) selon la revendication 2, **caractérisé en ce que** les moyens d'application (11, 15) comportent deux corps (15) de ce type, disposés face à face.

4. Mètre ruban à enroulement (1) selon la revendication 2 ou 3, **caractérisé en ce que** le corps (15) entoure un petit tube d'alimentation (11), qui comporte au moins une ouverture d'admission (13), s'engageant dans le réceptacle (9), et au moins une ouverture de sortie (13), disposée sur une surface périphérique (12).

5. Mètre ruban à enroulement (1) selon l'une quelconque des revendications 2 à 4, **caractérisé en ce que** le corps comporte une éponge (15).

6. Mètre ruban à enroulement (1) selon l'une quelconque des revendications 2 à 5, **caractérisé en ce que** le corps (15) comporte un feutre.

7. Mètre ruban à enroulement (1) selon la revendication 6, **caractérisé en ce que** le corps (15) comporte une lèvre en feutre.

8. Mètre ruban à enroulement (1) selon l'une quelconque des revendications précédentes, **caractérisé en ce que** le réceptacle (9) est fixé à l'extérieur contre le boîtier (2).

9. Fournitures complémentaires pour un mètre ruban à enroulement, qui est conçu pour mesurer des longueurs et des circonférences et comporte un boîtier (2) et un ruban de mesure (3) avec une graduation en unités de longueur, qui peut être enroulé à l'intérieur du boîtier (2) au moyen d'un dispositif d'enroulement et qui peut être guidé à travers une ouverture (5) du boîtier (2),
lesdites fournitures complémentaires comportant un réceptacle (9) pour un désinfectant liquide, un moyen de fixation (8) pour la fixation du réceptacle (9) contre le boîtier (2), et des moyens d'application (11, 15) qui sont conçus de telle sorte que, lorsque les fournitures complémentaires sont attachées au mètre ruban à enroulement et lorsque le réceptacle (9) contient un désinfectant, une partie du désinfectant est appliquée sur au moins une surface (16) du ruban de mesure (3) au moment où le ruban de mesure (3) passe à travers l'ouverture (5) du boîtier.

10. Fournitures complémentaires selon la revendication 9, **caractérisées en ce que** les moyens d'application (11, 15) comportent au moins un corps (15) qui, lorsque les fournitures complémentaires sont fixés sur le mètre ruban à enroulement, est disposé à proximité de l'ouverture (5) du boîtier de telle sorte qu'il vient en contact avec le ruban de mesure (3) au moment où le ruban de mesure (3) passe à travers l'ouverture (5) du boîtier, et qui est réalisé dans un matériau absorbant le liquide et est en liaison fluidique avec le réceptacle (9).

11. Fournitures complémentaires selon la revendication 10, **caractérisées en ce que** les moyens d'application (11, 15) comportent deux corps (15) de ce type, disposés face à face.

12. Fournitures complémentaires selon la revendication 10 ou 11, **caractérisées en ce que** le corps (15) entoure un petit tube d'alimentation (11), qui comporte au moins une ouverture d'admission (13), s'engageant dans le réceptacle (9), et au moins une ouverture de sortie (13), disposée sur une surface périphérique (12).

13. Fournitures complémentaires selon l'une quelconque des revendications 10 à 12, **caractérisées en ce que** le corps comporte une éponge (15).

14. Fournitures complémentaires selon l'une quelconque des revendications 10 à 13, **caractérisées en ce que** le corps (15) comporte un feutre.

15. Fournitures complémentaires selon la revendication 14, **caractérisées en ce que** le corps (15) comporte une lèvre en feutre.
